# EUROPÄISCHE PATENTANMELDUNG

(11) **EP 0 561 123 A1**
(43) Veröffentlichungstag der Anmeldung: **22.09.1993**
(21) Anmeldenummer: 93101173.8
(22) Anmeldetag: 27.01.1993
(51) Int. Cl.: A61M 5/145

(54) **Druckinfusionsapparat**

(30) Priorität: 17.03.1992 DE 4208482
(71) Anmelder: B. Braun Melsungen AG, D-34209 Melsungen (DE)
(72) Erfinder: Gerlach, Hans Josef, W-3538 Marsberg (DE); Steger, Jürgen, W-3582 Felsberg (DE)
(74) Vertreter: Selting, Günther, Dipl.-Ing.

(57) **Zusammenfassung**

Der Druckinfusionsapparat weist einen ersten Halter (11) zum Festlegen des Spritzenzylinders (14) einer Spritze (13) und einen linear bewegbaren zweiten Halter (18) auf. Der zweite Halter (18) ist ständig mit dem Motorantrieb (22) verbunden und von diesem nicht abkuppelbar. Die Schaltschritte des Schrittmotors (21) des Motorantriebs (20) werden in einem Vorwärts/Rückwärts-Zähler (32) gezählt, so daß die jeweilige momentane Position des zweiten Halters (18) anhand des Zählerstandes ermittelt werden kann.

## Beschreibung

Die Erfindung betrifft einen Druckinfusionsapparat zum Injizieren von Medikamenten oder anderen Flüssigkeiten in den Körper eines Patienten.

Bei Druckinfusionsapparaten zum Ausdrücken einer Spritze wird der Spritzenzylinder an einem ersten Halter festgelegt, während an dem Spritzenkolben ein zweiter Halter angreift, der von einem Motorantrieb linear bewegt wird. Durch Vorschieben des zweiten Halters wird die in der Spritze enthaltene Flüssigkeit ausgedrückt. Es ist häufig erforderlich, die momentane Position des zweiten Halters zu kennen, um eine Information über die bisher infundierte Flüssigkeitsmenge zu erhalten. Dies ist beispielsweise erforderlich, um die Infusionsrate nach Verabreichung einer bestimmten Flüssigkeitsmenge zu verändern oder um bei Vorhandensein eines vorbestimmten Restvolumens in der Spritze Voralarm zu erzeugen, damit die Spritze rechtzeitig gegen eine volle Spritze ausgewechselt werden kann, bevor sie leergelaufen ist.

Zum Einlegen einer Spritze in einen Druckinfusionsapparat muß bei den bekannten Apparaten der zweite Halter von dem Antrieb entkoppelt und manuell in eine Position geschoben werden, die es ermöglicht, die gefüllte Spritze einzulegen. Der Motorantrieb enthält somit eine Kupplung zum Abkuppeln und manuellen Bewegen des zweiten Halters.

EP 0 390 388 beschreibt einen Druckinfusionsapparat, bei dem der den Spritzenkolben vortreibende zweite Halter durch einen Motorantrieb angetrieben ist, der eine ausrückbare Kupplung enthält, damit der zweite Halter manuell bewegt werden kann. Im Vorschubweg des zweiten Halters ist ein Sensor angeordnet, der das Vorhandensein des zweiten Halters in einer Bezugsposition ermittelt. Auf das Sensorsignal hin zählt ein Mikroprozessor die nachfolgend an den Schrittmotor des Motorantriebs abgegebenen Impulse, um die momentane Position des zweiten Halters zu ermitteln. Wenn diese Impulszahl einen vorgegebenen Wert erreicht hat, wird Voralarm oder Endalarm ausgelöst. Der Sensor ist hierbei an einer Stelle in der Nähe des vorderen Endes des Weges des zweiten Halters angeordnet. Vor Erreichen des Sensors erfolgt die Bewegung des zweiten Halters ohne Wegmessung. Das in der Spritze befindliche Volumen ist bis zum Auslösen des Sensors unbekannt.

Der Erfindung liegt die Aufgabe zugrunde, einen Druckinfusionsapparat zu schaffen, der bei vereinfachtem Aufbau eine exakte Positionsbestimmung des zweiten Halters ermöglicht.

Die Lösung dieser Aufgabe erfolgt erfindungsgemäß mit den im Patentanspruch 1 angegebenen Merkmalen.

Bei dem erfindungsgemäßen Druckinfusionsapparat besteht ein ständiger Kraftschluß zwischen dem Antrieb und dem zweiten Halter. Die Position des zweiten Halters wird anhand der Steuersignale des Motors über den gesamten Verschiebeweg hinweg bestimmt. Die Stellung des zweiten Halters steht über die kraftschlüssige Antriebsverbindung jederzeit in einer genau festgelegten Beziehung zur Rotorstellung des des Motors. Auf diese Weise kann über die Steuersignale des Motors zu jedem Zeitpunkt und in jeder Position des zweiten Halters dessen genaue Stellung, und somit das genaue noch vorhandene Füllvolumen, ermittelt werden. Wichtig ist, daß die Positionsbestimmung nicht nur anhand von Vorwärtsbewegungen des zweiten Halters erfolgt, sondern daß auch Rückwärtsbewegungen dieses Halters mit einbezogen werden, damit die Positionsmessung nicht durch Bewegungen verfälscht wird, die vor dem Einlegen der Spritze in den Druckinfusionsapparat erfolgen. Ferner können auch Spritzen mit sehr kleinen Füllvolumen und kleinem Kolbenweg überwacht werden.

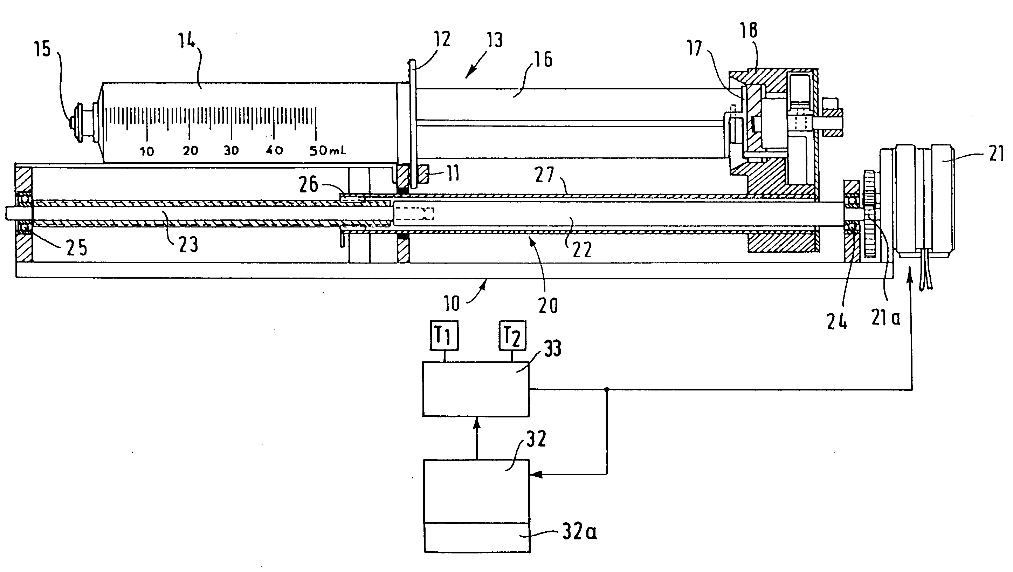

Im folgen wird unter Bezugnahme auf die einzige Figur der Zeichnung ein Ausführungsbeispiel der Erfindung näher erläutert.

Die Zeichnung zeigt eine Draufsicht des Druckinfusionsapparates, teilweise geschnitten, mit eingelegter Spritze.

Der Druckinfusionsapparat weist ein Gehäuse 10 auf, das in der Zeichnung durch einen horizontalen Balken angedeutet ist, an dem die einzelnen Komponenten befestigt sind. Am Gehäuse 10 ist ein erster Halter 11 angeordnet, an dem die Halteplatte 12 einer Spritze 13 festgelegt werden kann. Die Halteplatte 12 ist am rückwärtigen Ende des Spritzenzylinders 14 angebracht, der an seinem vorderen Ende einen Auslaßstutzen 15 für den Anschluß einer zum Patienten führenden Flüssigkeitsleitung aufweist. Der erste Halter 11 hält die Spritze 13 in definierter axialer Ausrichtung fest. In dem Spritzenzylinder 14 befindet sich der (nicht dargestellte) Spritzenkolben, der mit einer Kolbenstange 16 verbunden ist, welche aus dem rückwärtigen Ende des Spritzenzylinders herausragt. Am rückwärtigen Ende der Kolbenstange 16 ist eine querverlaufende Betätigungsplatte 17 vorgesehen.

Die Betätigungsplatte 17 ist beim Betrieb des Druckinfusionsapparates in den zweiten Halter 18 eingesetzt, der linear in Richtung auf den ersten Halter bewegt werden kann, um den Spritzenkolben im Spritzenzylinder 14 vorzuschieben und die Flüssigkeit aus dem Auslaßstutzen 15 herauszudrücken.

Der zweite Halter 18 wird von einem Motorantrieb 20 angetrieben. Dieser Antrieb weist einen am Gehäuse 10 befestigten Motor 21 auf, dessen Ausgangswelle 22 mit einer Schraubenspindel 23 fest verbunden ist. Die Ausgangswelle 22 und die Schraubenspindel 23 sind in Lagern 24,25 im Gehäuse gelagert. Auf dem Schraubengewinde der Schraubenspindel 23 sitzt eine Spindelmutter 26, die über ein die Ausgangswelle 22 umgebendes Rohr 27 mit dem zweiten Halter 18 fest verbunden ist. Der Motor 21 treibt über ein Untersetzungsgetriebe 21a die Ausgangswelle 21 an, wodurch die Schraubenspindel 23 gedreht wird. Dadurch wird die Spindelmutter 26, die gegen Drehung festgehalten ist, entlang der Schraubenspindel 23 linear bewegt. Diese Linearbewegung wird durch das Rohr 27 auf den zweiten Halter 18 übertragen.

Eine Steuereinrichtung 33, z.B. ein Mikroprozessor, steuert den Motor 21. Der Motor 21 ist hier ein Schrittmotor und die Steuereinrichtung 33 liefert die Antriebsimpulse für die inkrementale Weiterschaltung des Schrittmotors. Die Steuersignale werden ferner dem Zähleingang eines Zählers 32 zugeführt, der diese Zählimpulse zählt. Die Laufrichtung des Motors 21, und damit die Bewegungsrichtung des Halters 18, wird durch Drücken einer Tast T1 für "Vorlauf" oder einer Taste T2 für "Rücklauf" bestimmt. In Abhängigkeit davon, welche dieser beiden Tasten gedrückt ist, zählt der Zähler 32 entweder vorwärts oder rückwärts.

Der Rotor des Motors 21 ist über das Getriebe 21a und den Spindelantrieb 23,26,27 in ständiger Antriebsverbindung mit dem Halter 18. Dies bedeutet, daß sich in dem Antrieb keine Kupplung befindet.

Der jeweils aktuelle Zählerstand des Zählers 32 wird in einen nichtflüchtigen Speicher 32a eingespeichert, wo er auch bei Abschaltung des Gerätes oder bei Stromausfall festgehalten wird. Bei erneuter Inbetriebnahme des Geräts wird der der jeweiligen Position des zweiten Halters entsprechende Wert wieder aus dem Speicher 32a in den Zähler 32 zurückgegeben. Bei der Montage des Geräts wird der zweite Halter 16 in eine definierte Anfangsstellung gebracht, die einem bestimmten Zählerstand entspricht (Bezugsstellung). Von da an registriert der Zähler 32 alle Bewegungen des zweiten Halters, so daß dessen Position zu jeder Zeit durch den Zählerstand angegeben wird. Dabei ist wichtig, daß jede Bewegung des zweiten Halters 16 ausschließlich durch den Motorantrieb 20 erfolgt und daß keine manuelle Verschiebungen des Halters möglich sind. Der Antrieb ist rückwirkunsfrei, d.h. selbsthemmend, so daß eine Kraft, die auf den zweiten Halter einwirkt, weder diesen Halter noch den Motor verstellen kann. Ein Vorteil dieser Lösung besteht darin, daß ein Positionssensor zur Lieferung einer Referenzposition für die Impulszählung nicht erforderlich ist.

Wenn die Taste T1 für Vorlauf gedrückt wird, bewegt sich der Halter 18 in Richtung auf den Spritzenzylinder 14, wobei der Zähler 32 jeweils einen der momentanen Position des Halters 18 entsprechenden Wert enthält. Bei einem vorbestimmten Zählerstand des Zählers 32 kann Voralarm erzeugt werden. Ferner kann bei einem weiteren Zählerstand, der dem vollständigen Ausdrücken der Spritze 13 entspricht, Endalarm erzeugt werden.

Nach Betätigung eines nicht dargestellten Bedienelements bewirkt die Steuereinrichtung 33, daß der Halter 18 in seine rückwärtige Endstellung zurückgefahren wird, was dadurch erkannt wird, daß der Zähler 32 einen vorbestimmten Minimum-Zählerstand erreicht. Wenn eine neue Spritze eingesetzt worden ist, was durch einen das Vorhandensein des Spritzenzylinders 14 feststellenden Sensor erkannt wird, fährt die Steuereinrichtung 33 im Schnellgang nach vorne, bis der Halter 18 in Kontakt mit der Kolbenstange gelangt ist. Danach erfolgt der Vorschub mit der für den Infusionsvorgang vorgesehenen langsamen Vorschubgeschwindigkeit.

Ein wesentlicher Vorteil des Druckinfusionsapparates besteht darin, daß jederzeit die Position des Halters 18 bestimmt werden kann, ohne daß hierzu eine separate Wegmeßvorrichtung erforderlich ist.

## Patentansprüche

1. Druckinfusionsapparat mit einem ersten Halter (11) zum Festlegen des Zylinders (14) einer Spritze (13), einem zweiten Halter (18) zum Angreifen am rückwärtigen Ende der Kolbenstange (16) der Spritze (13), einem Motorantrieb (20) zum linearen Bewegen des zweiten Halters (18) relativ zum ersten Halter (11), und mit einer Steuereinrichtung (33), die aus von dem Motorantrieb (20) abgeleiteten Signalen die Position des zweiten Halters (18) ermittelt,
**dadurch gekennzeichnet,**
daß der zweite Halter (18) mit dem Motorantrieb (20) in permanenter, nicht trennbarer Antriebsverbindung steht und daß die Steuereinrichtung (33) die Position des zweiten Halters (18) über den gesamten Bewegungsweg durch Postitionsbestimmung der Rotorstellung des Motorantriebs feststellt.

2. Druckinfusionsapparat nach Anspruch 1, dadurch gekennzeichnet, daß der Motorantrieb (20) einen Schrittmotor aufweist, dessen von der Steuereinrichtung (33) gelieferte Antriebsimpulse in einem Vorwärts/Rückwärts-Zähler (32) vorzeichenrichtig gezählt werden, und daß der jeweils aktuelle Zählerstand des Zählers (32) in einem nicht-flüchtigen Speicher (32a) festgehalten wird.
